# EUROPEAN PATENT SPECIFICATION

(11) **EP 1 656 130 B1**
(45) Date of publication and mention of the grant of the patent: **30.07.2008**
(21) Application number: 04780570.0
(22) Date of filing: 10.08.2004
(51) Int. Cl.: A61K 31/201, A23K 1/16, A61P 19/02

(54) **OMEGA-3 FATTY ACIDS FOR THE TREATMENT OF CANINE OSTHEOARTHRITIS**
OMEGA-3-FETTSÄUREN ZUR BEHANDLUNG VON OSTEOARTHRITIS BEI HUNDEN
ACIDES GRAS OMEGA-3 POUR LE TRAITEMENT DE L'OSTEOARTHRITE DU CHIEN

(30) Priority: 11.08.2003 US 638832
(43) Date of publication of application: 17.05.2006
(73) Proprietor: Hill's Pet Nutrition Inc., Topeka, KS 66603 (US)
(72) Inventor: FRITSCH, Dale, A., Topeka, KS 66610 (US); JEWELL, Dennis, E., Lawrence, KS 66049 (US); SCHOENHERR, William, D., Hoyt, KS 66440 (US)
(74) Representative: Winckels, Johannes Hubertus F.
(86) International application number: PCT/US2004/025759
(87) International publication number: WO 2005/018630

(56) References cited:
- WO-A-99/04782
- US-A- 5 843 919
- RICHARDSON, D.C. ET AL.: "Nutritional Management of Osteoarthritis" VETERINARY CLINICS OF NORTH AMERICA, SMALL ANIMAL PRACTICE, vol. 27, no. 4, July 1997 (1997-07), pages 883-911, XP009040056 cited in the application
- MILLER, W.H. ET AL.: "Treatment of Dogs with Hip Arthritis with a Fatty Acid Supplement" CANINE PRACTICE, vol. 17, no. 6, 1992, pages 6-8, XP001203931
- BIERER, T.L.; BUI, L.M.: "Improvement of Arthritic Signs in Dogs Fed Greed-Lipped Mussel (Perna canaliculus)" AMERICAN SOCIETY FOR NUTRITIONAL SCIENCES. J. NUTR., vol. 132, 2002, pages 1634S-1636S, XP002306800
- BUDSBERG, S.: "Effects of fatty acid supplementation on the development of osteoarthritis in dogs: biochemical, clinical and radiographic evaluation" ABSTRACTS OF THE FIRST WORLD ORTHOPAEDIC VETERINARY CONGRESS, 2002, pages 56-57, XP002306801 MUNICH, GERMANY
- MUELLER, R.S.; ROSYCHUK, R.A.W.; JONAS, L.D.: "A retrospective study regarding the treatment of lupoid onychodystrophy in 30 dogs and literature review" JOURNAL OF THE AMERICAN ANIMAL HOSPITAL ASSOCIATION, vol. 39, no. 2, 2003, pages 139-150, XP009040039

## Description

Osteoarthritis is a degenerative joint disease commonly occurring in humans and in companion animals (Richardson et al., Vet. Clin. North Amer. Small Animal Practice 27:883-911, 1997; Curtis et al., Drug Disc. Today 9:165-172, 2004). The disease involves progressive deterioration of articular cartilage with minimal inflammation (Schoenherr et al. in Small Animal Clinical Nutrition 4th Ed., Hand et al. Eds., Walsworth Publishing Company, Marceline, MO, 2000, 907-921; Hedborn et al., Cell Mol. Life Sci 59:45-53, 2002; Pool, Front Biosci 4:D662-70, 1999). Management of osteoarthritis can include pharmacological treatments, surgery, nutraceutical administration and diet management. Such current management approaches have, however, focused on symptomatic relief and as such, they have not been entirely successful in disease management or in treating the underlying pathologies. Hence, there remains a continuing need for new approaches in managing osteoarthritis in humans and companion animals.

US-A-5,843,919 discloses to use a combination of glucosamine and an omega-3-fatty acid which combination is said to be effective in controlling symptoms of arthritis. Particularly, it is indicated that glucosamine is the chondroprotective agent and that the omega-3-fatty acid is an anti-inflammatory compound.

WO-99/04782 is directed to improving or maintaining the cell membrane essential fatty acid concentration. Particularly a synergistic effect is claimed for an essential fatty acid and a biocompatible disulphide.

In J, Nutr. vol 132, 3003, 1684S-1636S it is disclosed to use green-lipped mussels to improve arthritic signs in dogs.

The article in Canine Practice, vol. 17, no. 6, 1992, pages 6-8 attempts to disclose treating dogs with hip arthritis with fatty acid supplements.

### SUMMARY

Accordingly, the inventors herein have succeeded in discovering that administration of an effective amount of omega-3-fatty acids, in particular, Eicosapentaenoic acid (EPA) can provide a new approach for management of osteoarthritis in dogs.

Thus, the present invention relates to the use of EPA in the manufacture of a medicament for preventing or diminishing the degenerative process in joint cartilage in a dog having osteoarthritis or in the manufacture of a medicament for deceasing the likelihood of a dog developing osteoarthritis, wherein the medicament comprises EPA at a concentration of at least 0.2% by weight and the medicament. is to be administered to the dog in amount providing at least 27.5 mg EPA/kg body weight.
Preferably, the medicament comprises EPA at a concentration of at least 0.3% by weight.

In a preferred embodiment, the medicament can comprise a diet comprising EPA at a concentration of at least 0.2% by weight or, preferably, a concentration of at least 0.3% by weight.

The uses of the present invention restore a more nearly normal joint function in an osteoarthritic dog.

The present invention includes deceasing the likelihood of a dog developing osteoarthritis and restoring a more nearly normal joint function in an osteoarthritic dog by administering to the dog a composition comprising EPA in amount of about 37.5 mg/kg body weight, about 56.25 mg/kg body weight, about 75 mg/kg body weight or about 93.75 mg/kg body weight. The compositions used can be comprised by an animal food composition, an animal treat or an animal supplement.

In various embodiments, the uses can involve treating the osteoarthritic disease or reducing symptoms of the disease in the dog. Further, the uses can involve preventing development of the osteoarthritic disease in a dog or preventing or diminishing the appearance of symptoms of the disease in the dog.

The uses of the present invention can additionally be based upon compositions that further comprise omega-6 fatty acids in a total amount of not more than 3% by weight and/or a ratio of omega-6 fatty acids to omega-3 fatty acids of 0.2 to 1.1 and/or a ratio of omega-6 fatty acids to EPA of 1.0 to 12.5.

### DETAILED DESCRIPTION

This present use involves administration of omega-3 fatty acids, in particular, EPA in managing osteoarthritic discases and symptoms of such diseases in mammals and in particular, in dogs.

Omega-3 fatty acids also known as n-3 fatty acids, are a recognized group of polyunsaturated fatty carboxylic acids. In general, the omega-3 fatty acids contain 12-26 carbon atoms with methylene-interrupted double bonds. The physiologically more important omega-3 fatty acids are 18 - 22 carbons in length and straight chained. The n-3 fatty acids have a double bond between the 3 and 4 carbon atoms as measured from the methyl end of the molecule. Eicosapentaenoic acid (EPA), Docosahexaenoic acid (DHA) and alpha-feaolenic acid (ALA) are important n-3 fatty acids for managing osteoarthritis in mammals and EPA is particularly important in managing osteoarthritis in dogs. Derivatives of omega-3 fatty acids can also be used in managing osteoarthritis. Many types of derivatives are well known to one skilled in the art. Examples of suitable derivatives are esters, such as branched or unbranched and/or saturated or unsaturated C₁-C₃₀ cycloalkyl esters, in particular C₁-C₆ alkyl esters of omega-3 fatty acids, particularly EPA.

The omega-3 fatty acids, and in particular EPA, can be administered to a mammal, and in particular, to a dog, by any of many routes of administration, such as, for example, oral, intranasal, intravenous, subcutaneous and the like. The oral route is particularly suitable and EPA can be administered orally in a wet or dry diet, either incorporated therein or on the surface of any diet component, such as, by spraying, agglomerating, dusting or precipitating on the surface. It can be present in the nutritional diet per se or in a snack, supplement or a treat. It can also be present in the liquid portion of the diet such as water or another fluid. The EPA can be administered as a powder, solid or as a liquid including a gel. If desired the EPA can be orally administered in a nutraceutical or pharmaceutical dosage form such as a capsule, tablet caplet, syringe, and the like. Within the dosage form the EPA can be present as a powder or a liquid such as a gel. Any of the usual neutraceutical or pharmaceutical carriers can be employed such as water, glucose, sucrose and the like together with the EPA.

In certain embodiments, the present invention can involve EPA-diet compositions that are essentially free of DHA and/or ALA. Essentially free of DHA or ALA or mixtures thereof is intended to mean that either or both of DHA and ALA are substantially absent or that there am only small insignificant amounts of either or both of DHA or ALA present, for example, less than 0.1%, less than 0.03%, less than 0.01%, less than 0.03% or less than 0.001%. In embodiments that are essentially free of DHA and/or ALA, any amount of DHA and/or ALA present is at a concentration sufficiently low so thai no substantial effect is produced in an osteoarthritic dog on the disease of osteoarthritis, on the progression of osteoarthritis or on symptoms produced by the osteoarthritis.

EPA is effective against various forms of osteoarthritis as well as other forms of arthritis including rheumatoid arthritis.

The omega-3 fatty acid, EPA acts to prevent the development of the degenerative process in joint cartilege or to diminish the degenerative process and thereby improve joint in osteoarthritic dogs or in dogs that might otherwise develop osteoarthritis. This effect is in addition to an anti-inflammatory action of omega-3 fatty acids, which may be of less importance in canine osteoarthritis because a limited involvement of inflammation in the osteoarthritis,

Use of an *in vitro* explant procedure involving articular knee cartilage as shown in the examples below, demonstrated that EPA was the only omega-3 fatty acid to significantly decrease induced release of glycosaminoglycan (GAG) from the cartilage. With respect to prevention of joint damage from osteoarthritis a particular target group of pels, especially canines, arc those that would be in need of such preventative care as opposed to the general papulation. For example, pets, particularly large breed canines such as labrador retriever, rottweiler, german shepherd and the like are more susceptible to osteoarthritis as demonstrated by its greater occurrence in these pets. Additionally, pets above the age of six (6) years, particularly dogs, have a significantly greater occurrence of osteoarthritis. EPA can be additionally useful in treating canines and felines with osteoarthritis. Also present with the EPA can be other omega-3 fatty acids such as DHA and ALA as well as omega-6 fatty acids, all of which can be found in sources such as fish oils in relatively large quantities.

The quantity of EPA which should be employed can vary substantially. As shown in later examples, an actual dose response is observed - the greater the EPA, the greater the anti-arthritic effect. Generally, a minimum of at least 0.2 wt % based upon the quantity of a nutritious diet satisfying ordinary requirements of a canine or feline on a daily basis. For example, a specific amount can be employed in the usual nutrient food ration on a daily basis or the same daily quantity can be provided to the animal in a treat or supplement on a daily basis. Additionally, a combination of these methods or any other dosing means can be employed as long as the effective quantity of EPA is provided. The range of amounts of EPA includes at least 0.2%, at least 0.25%, at least 0.30%, at least 0.4%, at least 0.5%, at least 0.6% up to 2%, up to 2.25%, up to 2.5%, up to 3%, up to 4%, or up to 5% on a Weight basis. It should be noted that all wt % are on a dry matter basis (DMB). EPA is an omega-3 fatty acid. Generally, the ratios of the EPA or omega-3 to omega-6 fatty acid can vary significantly. In various embodiments, the omega-6:omega-3 ratio can be from 1.10 to 0.2 omega-6 to 1.0 omega-3 or from 1.08 to 0.42 omega-6 to 1.0 omega-3 and more particularly, 0.2, 0.25, 0.3, 0.4, 0.5, 0.6, 0.8, 1.0, or greater In various embodiments, the omega-6 to EPA ratio can be 12.5 to 1.0 omega-6 to 1.0 EPA, or 12.4 to 1.12 omega-6 to 1.0 EPA and more particularly, 0.2, 0.25, 0.3, 0.4, 0.4, 0.6, 0.8, 1.0, 1.5, 2.0, 2.5. 3, 4, 5, 6, 7.5, 10, 12.5 or greater. The arachidonic acid, AA, (an omega-6) to EPA ratio can be 0.28 to 0.01 AA to 1.0 EPA to 0.28 to 0.08 AA to 1.0 EPA and more particularly 0.01. 0.02, 0.04, 0.06, 0.08, 1.0, 1.5, 1.0, 1.5, 2.8 or greater.

The omega-3 fatty acid, and in particular EPA, can be administered in amounts calculated as mg/kg body weight. Thus for example, a 20 kg dog would be expected to consume 275 g of diet per day. Amounts of EPA in the diet of 0.2%, about 0.3%, about 4%, about 0.5% or about 0.6% by weight would amount to administering to the dog 27.5 mg/kg body weight, 41.25 mg/kg body weight, 55 mg/kg body weight, 68.75 mg/kg body weight or 82.5 mg/kg body weight respectively. More particularly, EPA can be administered in an amount of 20 mg/kg body weight, 28 mg/kg body weight, 30 mg/kg body weight, 40 mg/kg body weight, 41 mg/kg body weight, 50 mg/kg body weight, 55 mg/kg body weight, 60 mg/kg body weight, 69 mg/kg body weigh 70 mg/kg body weight, 80 mg/kg body weight, 82 mg/kg body weight, 90 mg/kg body weight, 100 mg/kg body weight, 120 mg/kg body weight, about 150 mg/kg body weight, or greater.

As indicated above, the EPA can be in the form of a food provided to the pet. Examples of such foods are regular diets providing all of the animal's nutrients, treats or supplements. The EPA can be provided in liquids or in pharmaceutical dosage forms such as capsules, tablets, pills, liquids or even parenterally administered such as through syringe. The most important aspect is that the pet be provided an effective amount of EPA to prevent or treat the osteoarthritis. In various embodiments, the route of administration can be oral and the EPA can be incorporated into a food. Foods are generally classified in the pet food industry as "wet" or "dry". A wet food has a relatively high amount of water and is usually present in a can or a container wherein air is substantially or totally excluded. Examples of such foods are "chunk and gravy", individual solid particles in the presence of a liquid gravy or a loaf type material which generally takes the shape of the receptacle. The dry food is generally a baked or preferably extruded material, the latter then cut into individual shaped portions, usually known as kibbles. EPA is readily incorporated into a wet food through conventional means. Encapsulation can be employed to protect the EPA from air oxidation in a dry diet. Additionally, use of antioxidants and nitrogen sweeps of packaging can also be employed. This is exemplified by US Patent No. 4,895,725 which has special emphasis on the micro-encapsulation of specific fish oils. Oils which have high levels of omega-3 fatty acids, are menhaden, salmon or cod.

### EXAMPLE 1

This example illustrates the release of glycosaminoglycan elicited by omega-3 fatty acids in cultured canine cartilage tissue.

Articular cartilage was obtained from left and right stifles (both femoral condyles and tibial plateau) of dogs. Cartilage explants cultured for 3 days in medium with 10% fetal bovine serum, then washed 3 times in serum free medium. Explants then cultured for 6 days in serum free medium containing 0,100 or 300 µg/ml n3 fatty acid (EPA, ALA or DHA). After this period in fatty acid medium, all explants were washed 3 times in fatty acid free/serum free medium. Explants were then cultured individually for 4 days in 1 ml of fatty acid and serum free medium containing no additives (C), 10 ⁻⁶M retinoic acid (RA) or 50 ng/ml oncostatin M (OSM). Note that not all treatments were possible on all dogs because of cartilage availability. The release of proteoglycan into the medium (µg/mg wet weight) was measured at the termination of culture. In the tables below, the mean and standard deviation of glycosaminoglycan (GAG) release for the triplicate cultures for each of the 4 dogs are given. In addition, the media lactate (µg/mg wt weight) concentrations were given for each treatment.

**TABLE 1.***

| **Dog 1 Treatment** | **GAG Mean** | **GAG Std Dev** | **n** | **GAG Min** | **GAG Max** | **Lactate Mean** | **Lactate Std Dev** |
|---|---|---|---|---|---|---|---|
| C | 1.363 | .497 | 3 | .849 | 1.84 | 26.07 | 33.7 |
| C+ carrier | 1.630 | .306 | 3 | 1.31 | 1.92 | 21.95 | 22.6 |
| C+ 100 EPA | 1.590 | .291 | 3 | 1.29 | 1.87 | 23.85 | 25.4 |
| C+ 300 EPA | 1.036 | .528 | 3 | .57 | 1.61 | NA** | |
| RA | 10.497 | 1.837 | 3 | 8.89 | 12.5 | 36 | 39.3 |
| RA+ carrier | 7.15 | 4.527 | 3 | 2. | 10.5 | 33.067 | 45.4 |
| RA+ 100 EPA | 8.677 | 1.999 | 3 | 6.61 | 10.6 | 29.367 | 34.8 |
| RA+ 300 EPA | 1.593 | 1.696 | 3 | .436 | 3.54 | 26.4 | 39.1 |
| OSM | 13.6 | 1.562 | 3 | 12.6 | 15.4 | 25.367 | 30.8 |
| OSM+ carrier | 14.25 | 6.44 | 3 | 7.35 | 20.1 | 27.4 | 33.8 |
| OSM+ 100 EPA | 6.293 | 2.301 | 3 | 4.34 | 8.8 | 33.567 | 52.5 |
| OSM+ 300 EPA | 2.167 | 1.93 | 3 | .93 | 4.39 | 20.05 | 23.8 |

| | | | | | | | |
|---|---|---|---|---|---|---|---|
| * GAG = glycosaminoglycan; C = serum free medium containing no additives, EPA = eicosapentaenoic acid; RA = retinoic acid; OSM = oncostatin M. ** not analyzed. | | | | | | | |

As shown in Table 1, significant decrease in GAG release occurred with 100 µg/ml EPA in OSM treated cultures and with 300 µg/ml in RA and OSM treated cultures. There was no significant decrease in media lactate concentrations with any dose of EPA.

**TABLE 2.***

| **Dog 2 Treatment** | **GAG Mean** | **GAG Std Dev** | **n** | **GAG Min** | **GAG Max** | **Lactate Mean** | **Lactate Std Dev** |
|---|---|---|---|---|---|---|---|
| C+ carrier | .503 | .422 | 3 | .127 | .96 | 22.8 | NA |
| C+ 100 EPA | .340 | .333 | 3 | .1 | .72 | 39.523 | 24.568 |
| C+ 300 EPA | .573 | .46 | 3 | .250 | 1.1 | 39.2 | 13.865 |
| OSM+ carrier | 11.7 | 5.11 | 3 | 7.1 | 17.2 | 26.9 | 4.766 |
| OSM+ 100 EPA | 5.25 | 3.002 | 3 | 2.19 | 8.19 | 21.7 | 9.838 |
| OSM+ 300 EPA | 2.83 | .229 | 3 | 2.66 | 3.09 | 16.233 | 3.602 |
| C+carrier | .973 | .222 | 3 | .84 | 1.23 | 17.4 | NA |
| C+ 100 DHA | .640 | .312 | 3 | .45 | 1 | 21 | 6.265 |
| C+ 300 DHA | .843 | .361 | 3 | .43 | 1.1 | 36.2 | NA |
| OSM+ carrier | 8.73 | .777 | 3 | 8.1 | 9.6 | 25.333 | 7.106 |
| OSM+ 100 DHA | 8.567 | 4.219 | 3 | 3.7 | 11.2 | 28.133 | 2.715 |
| OSM+ 300 DHA | 6.073 | 4.029 | 3 | 3.18 | 10.7 | 24.8 | 1.947 |
| C+ carrier | .821 | .684 | 3 | .193 | 1.55 | 15.567 | 1.955 |
| C+ 100 ALA | 1.12 | .089 | 3 | 1.05 | 1.22 | 28.4 | 13.718 |
| C+ 300 ALA | .993 | 1.104 | 3 | .14 | 2.24 | 41.667 | 14.958 |
| OSM+ carrier | 7.81 | 7.471 | 3 | .26 | 15.2 | 51.7 | 28.488 |
| OSM+ 100 ALA | 8.497 | 4.356 | 3 | 4.09 | 12.8 | 28.8 | 4.957 |
| OSM+ 300 ALA | 6.42 | 2.730 | 3 | 3.44 | 8.8 | 55.233 | 30.305 |

| | | | | | | | |
|---|---|---|---|---|---|---|---|
| * GAG = glycosaminoglycan; C = serum free medium containing no additives, EPA = eicosapentaenoic acid; RA = retinoic acid; OSM = oncostatin M. | | | | | | | |

As shown in Table 2, EPA but not ALA or DHA significantly decreased GAG release in OSM treated cultures. There was no significant effect on media lactate concentration by any dose of any of the fatty acids.

**TABLE 3.***

| **Dog 3 Treatment** | **GAG Mean** | **GAG Std Dev** | **n** | **GAG Min** | **GAG Max** | **Lactate Mean** | **Lactate Std Dev** |
|---|---|---|---|---|---|---|---|
| C+ carrier | 2.727 | .867 | 3 | 2.01 | 3.69 | 26.33 | 4.366 |
| C+ 100 ALA | 2.117 | .428 | 3 | 1.81 | 2.61 | 24.4 | 3.995 |
| C+ 100 DHA | 1.903 | .826 | 3 | 1.28 | 2.84 | 29.35 | 5.728 |
| C+ 100 EPA | 1.673 | .409 | 3 | 1.3 | 2.11 | 36.1 | NA |
| C+ 300 ALA | 2.447 | .321 | 3 | 2.14 | 2.18 | 20.75 | 7 |
| C+ 300 DHA | 1.55 | .73 | 3 | .73 | 2.13 | 28.4 | .566 |
| C+ 300 EPA | 1.567 | .387 | 3 | 1.3 | 2.01 | 10.525 | 10.854 |
| RA+carrier | 20.823 | .653 | 3 | 20.1 | 21.37 | 38.467 | 4.782 |
| RA+ 100 ALA | 20.44 | .903 | 3 | 19.4 | 21.02 | 43.233 | 2.281 |
| RA+ 100 DHA | 21.093 | 6.881 | 3 | 13.38 | 26.6 | 45.667 | 8 |
| RA+ 100 EPA | 16.223 | 6.654 | 3 | 8.61 | 20.93 | 41.533 | 2.515 |
| RA+ 300 ALA | 24.467 | 2.987 | 3 | 21.1 | 26.8 | 44.733 | 4.821 |
| RA+ 300 DHA | 19.457 | 2.389 | 3 | 17.28 | 22 | 47.967 | 9.139 |
| RA+ 300 EPA | 1.537 | .618 | 3 | 1.08 | 2.24 | NA | NA |
| OSM+ carrier | 12.773 | 5.845 | 3 | 6.36 | 17.8 | 37.867 | 11.547 |
| OSM- 100 ALA | 22.033 | 4.596 | 3 | 18.4 | 27.2 | 32.767 | 1.815 |
| OSM- 100 DHA | 11.667 | 6.007 | 3 | 5.5 | 17.5 | 32.267 | 11.467 |
| OSM-100 EPA | 17.85 | 2.051 | 3 | 16.4 | 19.3 | 39.05 | 11.526 |
| OSM- 300 ALA | 23.467 | 3.102 | 3 | 20.3 | 26.5 | 34.033 | 1.38 |
| OSM- 300 DHA | 11.630 | 5.069 | 3 | 6.79 | 16.9 | 30.0 | 5.963 |
| OSM- 300 EPA | 8.1 | 6.767 | 3 | 3.79 | 15.9 | 21.467 | 1.93 |

| | | | | | | | |
|---|---|---|---|---|---|---|---|
| * GAG = glycosaminoglycan; C = serum free medium containing no additives, EPA = eicosapentaenoic acid; RA = retinoic acid; OSM = oncostatin M. | | | | | | | |

As shown in Table 3, none of the fatty acids significantly altered GAG release from RA- or OSM-stimulated cartilage in this particular animal. There was no change in media lactate associated with any dose of any fatty acid.

**TABLE 4.***

| **Dog 4 Treatment** | **GAG Mean** | **GAG Std Dev** | **n** | **GAG Min** | **GAG Max** | **Lactate Mean** | **Lactate Std Dev** |
|---|---|---|---|---|---|---|---|
| C+ carrier | 1.96 | .533 | 3 | 1.51 | 2.55 | 22.933 | 4.75 |
| C+ 100 ALA | 2.103 | .107 | 3 | 1.98 | 2.17 | 20.533 | 3.478 |
| C+ 100 DHA | 2.343 | .331 | 3 | 2 | 2.66 | 19.1 | 2.352 |
| C+ 100 EPA | 2.687 | .996 | 3 | 1.72 | 3.71 | 23 | 6.183 |
| C+ 300 ALA | 1.533 | 1.244 | 3 | .13 | 2.5 | 29.167 | 22.074 |
| C+ 300 DHA | 2.307 | .361 | 3 | 1.93 | 2.65 | 24.933 | 3.4 |
| C+ 300 EPA | 2.1 | .455 | 3 | 1.64 | 2.55 | 24.767 | 13.004 |
| RA+ carrier | 14.113 | 3.89 | 3 | 9.64 | 16.7 | 34.533 | 12.368 |
| RA+ 100 ALA | 12.547 | 6.348 | 3 | 5.94 | 18.6 | 39.933 | 11.594 |
| RA+ 100 DHA | 11.28 | 7.123 | 3 | 4.79 | 18.9 | 25.6 | 11.766 |
| RA+ 100 EPA | 14.393 | 2.9 | 3 | 11.23 | 16.93 | 32.967 | 4.219 |
| RA+ 300 ALA | 14.093 | 6.138 | 3 | 8.98 | 20.9 | 59.367 | 31.166 |
| RA+ 300 DHA | 11.3 | 6.815 | 3 | 3.5 | 16.1 | 25.333 | 11.684 |
| RA+ 300 EPA | 9.093 | 1.316 | 3 | 8.26 | 10.61 | 25.1 | 4.67 |
| OSM+ carrier | 16.083 | 3.544 | 3 | 12.05 | 18.7 | 31.2 | 5.991 |
| OSM+ 100 ALA | 11.7 | 2.19 | 3 | 9.43 | 13.8 | 26.333 | 9.25 |
| OSM+ 100 DHA | 24.967 | 3.262 | 3 | 21.2 | 26.9 | 36.833 | 5.066 |
| OSM+ 100 EPA | 15.883 | 4.316 | 3 | 11.95 | 20.5 | 27.237 | 6.34 |
| OSM+ 300 ALA | 19.557 | 3.909 | 3 | 15.5 | 23.3 | 26.667 | 6.099 |
| OSM+ 300 DHA | 16.4 | 6.27 | 3 | 9.4 | 21.5 | 36.233 | 20.342 |
| OSM+ 300 EPA | 13.493 | 5.752 | 3 | 7.54 | 19.02 | 27.8 | 2.722 |

| | | | | | | | |
|---|---|---|---|---|---|---|---|
| * GAG = glycosaminoglycan; C = serum free medium containing no additives, EPA = eicosapentaenoic acid; RA = retinoic acid; OSM = oncostatin M. | | | | | | | |

As shown in Table 4, EPA at 300 µg/ml, but not any other fatty acid at any dose, significantly decreased GAG release from RA treated cultures. There was a significant decrease in media lactate concentration in control. RA- and OSM- treated cultures with the 300 µg/ml OSM pre-treatment.

### EXAMPLE 2

This example illustrates the incorporation of n-3 fatty acids into canine chondrocyle membranes.

The majority of these experiments were performed using monolayer cultures, however, in a single experiment, the incorporation of fatty acids into explant cultures of canine cartilage was analyzed.

### Monolayer Cultures

Over 24 or 48 hours there was no incorporation of the 18:3 n-3 fatty acid ALA into chondrocyte membranes from two dogs. The % 18:3 n-3 in chondrocytes incubated in medium alone was < 1 out of 5 (range = 0.3-0.9%) and after 24 or 48 hours of incubation with 100 or 300 µg/ml ALA this percentage had not significantly changed (range = 0.3-2.5%).

Over 48 hours there was significant incorporation of the 20:5 n-3 fatty acid EPA into chondrocyte membranes from one dog. The % 20:5 n-3 increased from <1% (range = 0.2-0.6%) to approximately 7% (range = 5.6-8%) when cultures were treated with 100 or 300 µg/ml EPA for 48 hours. The incorporation was not different when cultures were performed in the presence or absence of 5% FCS.

Over 48 hours there was significant incorporation of the 20:5 n-3 fatty acid EPA but not the 18:3 n-3 fatty acid ALA into chondrocyte membranes from one dog (doses of 300 µg/ml for each fatty acid). The % 20:5 n-3 increased from <1% to approximately 15%.

Over 3 or 6 days there was significant incorporation of the 20:5 n-3 fatty acid EPA into chondrocyte membranes from one dog (dose of 300 µg/ml EPA). The % 20:5 n-3 increased from <1% to 16-18% with no difference between 3 and 6 days incubation.

Explant Culture

Over 6 days there was apparent incorporation of the 20:5 n-3 fatty acid EPA, but not the 18:3 n-3 DHA or the n-6 fatty acid AA (arachadonic acid) into cartilage explants from one dog (dose of 300 µg/ml for each fatty acid). The % n-3 20:5 increased from 0% (none detectable) to approximately 2%.

These data indicated that EPA, but no other n-3 fatty acid was incorporated into canine chondrocyte membranes in either monolayer or explant cultures.

### EXAMPLE 3

This example illustrates the effect of n-3 Fatty Acids on Canine Chondrocyte Metabolism.

To assess the potential effect of n-3 fatty acids on protein and proteoglycan metabolism in canine cartilage, cultures were set up as described in Example 1 except for the final 4 days of culture, no catabolic stimuli were added (i.e., all "control" cultures). During the final 24 hours of culture (1) ³⁵SO₄, or (ii) ³⁵S-methionine and ³⁵S-cysteine were added to the medium to radiolabel newly synthesized proteoglycans and proteins, respectively. The incorporation of radiolabel into the cartilage matrix was measured at the termination of culture. No attempt was made to quantitate loss of radiolabelled material from the cartilage over the 24-hour labeling period. The mean and standard deviation of the incorporation of ³⁵SO₄ ("PG") or ³⁵S-methionine and ³⁵S-cysteine ("PROT") as DPM/mg wet weight are shown in Table 5 below.

**TABLE 5.***

| **Treatment** | **PG Mean** | **PG Std Dev** | **N** | **PROT Mean** | **PROT Std Dev** |
|---|---|---|---|---|---|
| Carrier | 292.667 | 53.144 | 3 | 574.333 | 198.336 |
| 100 ALA | 246.333 | 100.779 | 3 | 503.667 | 184.218 |
| 100 DHA | 156.0 | 82.529 | 3 | 503.667 | 81.365 |
| 100 EPA | 537.333 | 161.81 | 3 | 442.0 | 72.746 |
| 300 ALA | 443.0 | 205.385 | 3 | 393.667 | 34.962 |
| 300 DHA | 123.333 | 38.24 | 3 | 564.333 | 220.048 |
| 300 EPA | 275.667 | 161.661 | 3 | 504.0 | 44.542 |

| | | | | | |
|---|---|---|---|---|---|
| * PG = incorporation of ³⁵SO₄ in DPM/mg wet weight; PROT = incorporation of ³⁵S-methionine and ³⁵S-cysteine as DPM/mg wet weight; EPA = eicosapentaenoic acid; DHA = docosahexaenoic acid; ALA = alpha-lenolenic acid. | | | | | |

As shown in Table 5, there was no significant effect of any n-3 fatty acid on protein synthesis and incorporation into the matrix. EPA at 100 µg/ml significantly increased proteoglycan synthesis and incorporation. No other dose or fatty acid significantly altered proteoglycan synthesis and incorporation into the cartilage matrix.

Reverse transcription-PCR was used to measure the mRNA message expression levels of matrix proteinases (aggrecanases -1 and -2), cyclooxygenases -1 & - 2, lipoxygenases - 5 and 12, and potential autocrine cytokines and their receptors (e.g. IL-1, IL-6 and TNF).

The results of this study found that aggrecanase-1 and aggrecanase-2 mRNA messages were expressed in "normal" canine cartilage tissue. In addition, some dogs expressed mRNA message of cyclooxygenase-2 (COX-2) message although there were no signs of joint pathology in these animals. This enabled monitoring the effects of n-3 and n-6 fatty acid supplementation on mRNA expression of aggrecanases and COX-2 in unstimulated canine articular cartilage explants. EPA was the only fatty acid able to reduce the mRNA message for the degradative enzymes, aggrecanase-1 and aggrecanase-2, in canine articular cartilage. This demonstrated the ability of EPA to "turn off" the genes responsible for cartilage degradation.

### EXAMPLE 4

This study illustrates the effects of omega-3 fatty acids in canine osteoarthritis clinical studies.

Three clinical studies were conducted in pet dogs clinically diagnosed with osteoarthritis. Veterinary general practitioners and orthopedic specialists enrolled client owned dogs that met a specific eligibility criteria. All patients were required to: have radiographic evidence of osteoarthritis with measurable clinical manifestations of disease, based on historical accounts by pet owners and physical examinations by veterinarians; be otherwise healthy and free of concurrent diseases based on physical exam, CBC, blood chemistry, and urinalysis; maintain regimen of therapy if receiving medications or supplements prescribed for osteoarthritis during the 30 days prior to enrolling in the study.

The following measurements were made.

Serum fatty acid profile: This was determined by a gas chromatography method involving extraction of fatty acids by chloroform and methanol mixture (2:1), methylation using boron trifluride-methanol (BF₃:MeOH) reagent followed by flame ionization detection (FID). Fatty acid methyl esters were identified by comparison of retention times with those of known standards and quantitated using an internal standard.

Veterinary clinical evaluation: Veterinarians conducted both a physical exam and a clinical evaluation of the patient's osteoarthritic condition during the screening phase and at the conclusion of each of the feeding intervals over the course of the clinical trial. Veterinarians assessed the severity of five osteoarthritic parameters: lameness, reluctance to bear weight, reduction in range of motion, reluctance to hold up contra-lateral limb, and pain on palpation of the joint. Changes in severity scores for these individual parameters were measured over the duration of the feeding period. A comprehensive veterinary clinical assessment of the impact of dietary intervention on the osteoarthritic condition of patients was derived by combining the changes in severity scores for all five individual parameters.

Pet owner subjective evaluation: Pet owners were required to complete an enrollment questionnaire prior to participating in the study and additional questionnaires at the conclusion of each of the feeding intervals over the course of the clinical trial.

**·** Enrollment questionnaire - pet owners rated the observed frequency and severity of the most common signs of canine osteoarthritis including difficulty rising from rest, limping, stiffness, soreness when touched, lagging behind during walks, yelping or whimpering in pain, aggressive behaviors, difficulty in running, difficulty in walking, difficulty in climbing steps, difficulty in jumping, difficulty in playing, impaired mobility, and overall activity level. In addition, owners rated the overall osteoarthritic condition of their pet.

**·** Feeding questionnaire - pet owners rated both the frequency and change in severity of the signs of canine osteoarthritis which were benchmarked during enrollment. In addition, the pet owners rated the severity of their animal's pain associated with osteoarthritis.

Force plate gait analysis: Dogs were evaluated at each respective institution using a computerized biomechanics force plate at day 0, 6 weeks, and 12 weeks. The plate was mounted centrally in and flush with the surface of a 10 m walkway. A handler trotted dogs across the force plate and an observer evaluated each pass across the plate to confirm foot-strikes and gait. A trial was considered valid if there were distinct ipsilateral fore foot and hind foot strikes while the dog was trotted across the force plate at a velocity of 1.7 to 2.0 m/s, with an acceleration variation of -0.5 to 0.5 ms². During each trial, the dog's forward velocity was measured, using a millisecond timer and two photoelectric switches. Each trial was videotaped for review and confirmation of valid foot-strikes. Care was taken to ensure that the dog triggered the timer and that a consistent speed (as perceived by the handler and observer) was maintained across the plate during each trial.

Five valid trials for each test period were obtained for each affected limb and each ipsolateral limb of each dog. Orthogonal ground reaction forces of peak vertical force, vertical impulse, braking and propulsive peak forces, and braking and propulsion impulses were measured and recorded by a specialized software program. (Acquire, Sharon Software, DeWitt, MI), All forces were normalized with respect to body weight in kilograms. Data from the valid trial for each limb were averaged to obtain a mean value for each force or impulse at each time period.

Ground reaction force data were compared between treatment and placebo groups as a percentage difference between lame and ipsolateral limbs at each time period. Percentage change of ground force data on the lame limb were compared at the beginning and end of the feeding period.

### STUDY #1

A canine study was conducted to evaluate the dietary effect of feeding high levels of n-3 fatty acids to dogs diagnosed with osteoarthritis. Eighteen veterinary general practitioners were recruited to enroll patients in the study. A total of 131 dogs were randomly assigned to two dietary treatments and fed for 180 days. The test and control foods had similar macronutrient profile, but were significantly different fatty acid composition (Table 6). The test diet contained high levels of ALA, EPA, and DHA, and was formulated with a low n-6 / n-3 ratio. The control diet was a leading selling commercially available dog food, with typical levels of n-3 fatty acids and n-6 /n-3 ratio characteristic for the industry.

**TABLE 6.***

| **Dietary Nutrient** | **Control Food (%)** | **Test Food (%)** |
|---|---|---|
| Protein | 23.2 | 19.9 |
| Fat (total) | 13.9 | 13.6 |
| CHO₂ (NFE) | 54.7 | 53.3 |
| C18:3 n-3 (ALA) | 0.12 | 2.8 |
| C20:4 n-6 (AA) | 0.03 | 0.06 |
| C20:5 n-3 (EPA) | <0.01 | 0.38 |
| C22:6 n-3 (DHA) | <0.01 | 0.31 |
| Sum n-6 | 1.99 | 2.53 |
| Sum n-3 | 0.09 | 3.48 |
| n6/n3 ratio | 22.8 | 0.7 |

| | | |
|---|---|---|
| *NFE = Soluble carbohydrate content as Nitrogen Free Extract; ALA = alpha-lenolenic acid; AA = arachidonic acid; EPA = eicosapentaenoic acid; DHA = docosahexaenoic acid. | | |

Serum fatty acids and pet owner evaluations were recorded at 0, 45, 90 and 180 days. Serum fatty acid profiles were significantly modulated by the test food. The test group had significantly higher concentrations of n-3 fatty acids (P<0.01), specifically EPA, DHA, a-ALA, significantly lower concentrations of AA (P<0.01), and significantly lower n-6:n-3 ratios (P<0.01) as compared to the control group at the conclusion of each feeding interval (Table 7). The test group showed significant improvements for rising from rest, running, and playing at day 45 and walking at days 90 and 180 as compared to the control group based on pet owner observations (P<0.05), even in the presence of a strong placebo effect (Table 8).

**TABLE 7.***

| **Canine Mean Serum Fatty Acid Levels (mg/dl)** | | | | | |
|---|---|---|---|---|---|
| | **Group** | **Day 0** | **Day 45** | **Day 90** | **Day 180** |
| C 18:3 n-3 (α - ALA) | Control | 1.10 | 0.89 | 0.52 | 0.53 |
| | Test | 1.05 | 5.61 | 6.51 | 7.13 |
| C20:4 n-6 (AA) | Control | 71.35 | 66.34 | 68.03 | 68.21 |
| | Test | 64.32 | 45.90 | 46.13 | 42.65 |
| C20:5 n-3 (EPA) | Control | 1.14 | 0.90 | 0.67 | 0.93 |
| | Test | 1.28 | 16.28 | 18.64 | 19.94 |
| C22:6 n-3 (DHA) | Control | 2.67 | 2.03 | 1.70 | 1.98 |
| | Test | 2.93 | 11.31 | 12.24 | 12.17 |
| Sum n-6 | Control | 141.08 | 138.72 | 137.85 | 140.28 |
| | Test | 130.85 | 118.87 | 128.71 | 123.99 |
| Sum n-3 | Control | 4.95 | 3.84 | 2.93 | 3.51 |
| | Test | 5.36 | 33.20 | 37.39 | 39.24 |
| n-6:n-3 ratio | Control | 33.33 | 37.95 | 51.59 | 51.39 |
| | Test | 33.90 | 7.47 | 8.63 | 6.92 |

| | | | | | |
|---|---|---|---|---|---|
| * ALA = alpha-lenolenic acid; AA = arachidonic acid; EPA = eicosapentaenoic acid; DHA = docosahexaenoic acid.* | | | | | |

**TABLE 8.***

| **Pet Owner Observed Change in Severity of Osteoarthritis*** | | | | | | | |
|---|---|---|---|---|---|---|---|
| | | **Day 0-45** | | **Day 45-90** | | **Day 90-180** | |
| Osteoarthritic Sign | Group | Mean | P Value | Mean | P Value | Mean | P Value |
| Rising from rest | Control | 1.77 | .041 | 1.77 | nsd** | 1.93 | nsd** |
| | Test | 1.56 | | 1.84 | | 1.91 | |
| Running | Control | 1.81 | .037 | 1.83 | nsd** | 1.94 | nsd** |
| | Test | 1.56 | | 1.71 | | 1.91 | |
| Walking | Control | 1.71 | nsd** | 2.00 | .018 | 2.19 | .002 |
| | Test | 1.69 | | 1.71 | | 1.75 | |
| Playing | Control | 1.83 | .008 | 1.90 | nsd** | 2.06 | nsd** |
| | Test | 1.50 | | 1.78 | | 1.97 | |

| | | | | | | | |
|---|---|---|---|---|---|---|---|
| * Osteoarthritis severity rating scale: 1 = better, 2 = no change, 3 = worsened. ** nsd = no significant difference. | | | | | | | |

### STUDY #2

A canine study was conducted to evaluate the dietary effect of feeding high levels of n-3 fatty acids to dogs diagnosed with osteoarthritis. Two veterinary orthopedic specialists enrolled patients in the study. A total of 38 dogs were randomly assigned to two dietary treatments and feed for 90 days. The test and control diets were manufactured from the same lots of foods as described above (Table 6).

Serum fatty acids, force plate gait analysis, and veterinary clinical assessments were recorded at 0, 45, and 90 days. Serum fatty acid profiles were significantly modulated by the test food. The test group had significantly higher serum concentrations of n-3 fatty acids (P<0.01), specifically EPA, DHA, ALA, significantly lower concentrations of AA at day 90 (P<0.01), and significantly lower n-6:n-3 ratios (P<0.01) as compared to the control group at the conclusion of each feeding interval (Table 9).

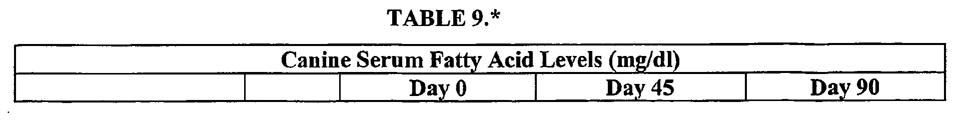

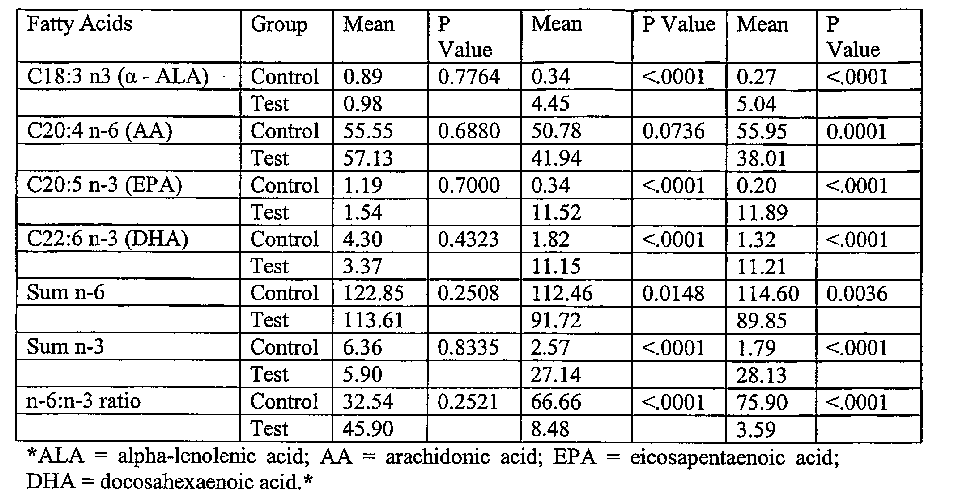

A biomechanical assessment of the dogs' most severe osteoarthritic limb was objectively evaluated using force plate gait analysis (Table 10). Vertical peak force is the key parameter measured to determine weight bearing of the affected limb. There was no significant change in mean vertical peak force over the duration of the 90 day feeding for the control group (P=0.91), while there was a significant increase in mean vertical peak force over time for the test group (P=0.01). The percent mean change in vertical peak force was also significantly different between groups (P<0.05), indicating that the test group increased weight bearing in the affected limb, while the control group displayed no change in weight bearing over the course of the study. Weight bearing ability can also be represented by displaying the frequency distribution of percent change in vertical peak for each dietary group. Only 31 % of animals in the control group showed improvement in weight bearing after the 90 day feeding, while 82% of the dogs in the test group increased weight bearing over the course of the study.

**TABLE 10.**

| **Vertical Peak Force** | | | | | | | | |
|---|---|---|---|---|---|---|---|---|
| | **Day 0** | | **Day 90** | | **Change (Day 0 - 90)** | | | |
| Group | Mean | P Value | Mean | P Value | Mean Change | Mean = 0 Pr > 1 t 1 | % Mean Change | Pr > 1 t 1 |
| Control | 72.80 | 0.5981 | 72.63 | 0.9323 | -0.17 | 0.9144 | -0.58 | 0.0443 |
| Test | 69.51 | | 73.21 | | 3.71 | 0.0103 | 5.35 | |

The subjective clinical evaluations performed by the veterinary orthopedic surgeons provided additional support for the efficaciousness of the test diet. Based upon the comprehensive veterinary clinical assessment, a significantly greater percent of dogs were evaluated as improved that consumed the test food as compared to dogs that consumed the control food (P<0.05). The veterinary specialists also observed a greater percent of dogs in the test group displaying a reduction in pain on palpation of the joint as compared to the control group (P=0.05).

### STUDY #3

A canine study was conducted to determine the dose effect of feeding high levels of n-3 fatty acids to dogs diagnosed with osteoarthritis. Twenty-eight veterinary general practitioners enrolled patients in the study. A total of 177 dogs were randomly assigned to three dietary treatments and fed for 90 days. Approximately two-thirds of the dogs participating in the study were receiving medications and / or supplements prescribed for treating osteoarthritis, in addition to consuming the therapeutic diets being evaluated. The three test foods had similar macronutrient profiles, but varied in composition of EPA and DHA, with variable A containing the lowest levels and variable C containing the highest levels (Table 11).

**TABLE 11.***

| | **Test Variable %** | | |
|---|---|---|---|
| **Dietary Nutrient** | **A** | **B** | **C** |
| Protein | 19.97 | 19.51 | 19.37 |
| Fat (total) | 13.78 | 15.34 | 19.55 |
| CHO₂ (NFE) | 53.92 | 52.34 | 47.66 |
| C18:3 n-3 (ALA) | 2.65 | 1.18 | 1.10 |
| C20:4 n-6 (AA) | 0.11 | 0.18 | 0.24 |
| C20:5 n-3 (EPA) | 0.50 | 1.18 | 1.69 |
| C22:6 n-3 (DHA) | 0.34 | 0.80 | 1.15 |
| Sum n-6 | 2.70 | 2.45 | 2.14 |
| Sum n-3 | 3.54 | 3.53 | 4.52 |
| n6 / n3 ratio | 0.76 | 0.7 | 0.47 |

| | | | |
|---|---|---|---|
| *NFE = Soluble carbohydrate content as Nitrogen Free Extract; ALA = alpha-lenolenic acid; AA = arachidonic acid; EPA = eicosapentaenoic acid; DHA = docosahexaenoic acid. | | | |

Serum fatty acids, pet owner evaluations, and veterinary clinical assessments were recorded at 0, 21, 45, and 90 days. Serum fatty acid profiles were significantly modulated by all dietary variables. The dogs fed test variables B & C had significantly higher serum concentrations of n-3 fatty acids (P<0.01), specifically EPA, DHA, ALA, significantly lower concentrations of n-6 fatty acids, specifically AA (P<0.01), and significantly lower n-6:n-3 ratios (P<0.01) as compared to the dogs feed test variable A at the conclusion of each feeding interval (Table 12).

**TABLE 12***

| **Canine Serum Fatty Acid Levels (mg/dl)** | | | | | |
|---|---|---|---|---|---|
| | | **Day 0** | **Day 21** | **Day 45** | **Day 90** |
| **Fatty Acids** | **Group** | **Mean** | **Mean** | **Mean** | **Mean** |
| C18:3 n-3 (ALA) | A | 1.34 | 5.65 | 5.29 | 5.63 |
| | B | 1.29 | 3.36 | 3.99 | 3.82 |
| | C | 1.25 | 2.92 | 3.32 | 3.29 |
| C20:4 n-6 (AA) | A | 76.37 | 51.10 | 47.54 | 47.77 |
| | B | 73.15 | 41.55 | 38.94 | 37.0 |
| | C | 70.05 | 37.35 | 36.86 | 34.73 |
| C20:5 n-3 (EPA) | A | 1.32 | 18.74 | 18.51 | 19.26 |
| | B | 1.54 | 26.14 | 29.87 | 30.03 |
| | C | 1.85 | 34.42 | 35.71 | 39.04 |
| C22:6 n-3 (DHA) | A | 3.50 | 13.75 | 13.84 | 13.88 |
| | B | 4.72 | 18.47 | 19.98 | 20.16 |
| | C | 3.91 | 21.01 | 21.47 | 22.49 |
| Sum n-6 | A | 150.38 | 114.38 | 110.12 | 112.70 |
| | B | 143.93 | 93.83 | 95.87 | 92.10 |
| | C | 139.97 | 79.71 | 82.65 | 80.74 |
| Sum n-3 | A | 6.16 | 38.14 | 37.65 | 38.77 |
| | B | 7.55 | 47.96 | 53.84 | 54.01 |
| | C | 7.01 | 58.35 | 60.50 | 68.83 |
| n-6:n-3 ratio | A | 29.99 | 5.65 | 3.48 | 3.75 |
| | B | 28.09 | 3.36 | 1.92 | 1.79 |
| | C | 32.30 | 2.92 | 2.02 | 1.73 |

| | | | | | |
|---|---|---|---|---|---|
| *ALA = alpha-lenolenic acid; AA = arachidonic acid; EPA = eicosapentaenoic acid; DHA = docosahexaenoic acid. | | | | | |

Pet owners reported improvements in 13 of 14 individual osteoarthritic signs for dogs consuming any of the dietary variables for 21 days (Table 13). Additionally, pet owners reported a decrease in severity for 13 of 14 individual osteoarthritic signs for dogs consuming any of the dietary variables for 90 days (Table 14). Pet owners also reported a significant reduction in the frequency of observable osteoarthritic signs after the dogs consumed any of the dietary variables for 90 days (Table 15).

**TABLE 13.**

| **Pet Owner Observed Improvements in Osteoarthritic Signs (Day 0-21)** | | | | | | | |
|---|---|---|---|---|---|---|---|
| Osteoarthritic Sign | Die t | Mean | Mean = 0 Pr > 1t 1 | Osteoarthritic Sign | Diet | Mean | Mean = 0 Pr > 1t 1 |
| Rising from rest | A | -0.439 | 0.0002 | Running | A | -0.524 | 0.0004 |
| | B | -0.738 | <.0001 | | B | -0.682 | <.0001 |
| | C | -0.763 | <.0001 | | C | -0.674 | <.0001 |
| Limping | A | -0.720 | <.0001 | Walking | A | -0.553 | 0.0007 |
| | B | -0.731 | <.0001 | | B | -0.750 | <.0001 |
| | C | -0.837 | <.0001 | | C | -0.667 | <.0001 |
| Stiffness | A | -0.537 | <.0001 | Stair Climbing | A | -0.449 | 0.0012 |
| | B | -0.783 | <.0001 | | B | -0.667 | <.0001 |
| | C | -0.627 | <.0001 | | C | -0.723 | <.0001 |
| Soreness | A | -0.750 | 0.0005 | Jumping | A | -0.362 | 0.0049 |
| | B | -0.800 | 0.0002 | | B | -0.600 | <.0001 |
| | C | -0.379 | 0.0451 | | C | -0.542 | <.0001 |
| Lagging behind on walks | A | -0.564 | 0.0004 | Playing | A | -0.622 | <.0001 |
| | B | -0.909 | <.0001 | | B | -0.763 | <.0001 |
| | C | -0.531 | 0.0022 | | C | -0.487 | 0.0014 |
| Pain | A | -0.476 | 0.0245 | Impaired Mobility | A | -0.528 | 0.0005 |
| | B | -0.478 | 0.0184 | | B | -0.700 | <.0001 |
| | C | -0.889 | 0.0002 | | C | -0.564 | 0.0001 |
| Aggression | A | 0.000 | 1.0000 | Activity Level | A | -0.745 | <.0001 |
| | B | -0.313 | 0.1050 | | B | -0.857 | <.0001 |
| | C | -0.429 | 0.1401 | | C | -0.865 | <.0001 |

| | | | | | | | |
|---|---|---|---|---|---|---|---|
| The above "p" values refer to the mean change from day 0 to day 21. | | | | | | | |

**TABLE 14.**

| **Difference in Pet Owners Severity Rating (day 0-90)** | | | | | | | | |
|---|---|---|---|---|---|---|---|---|
| **Osteoarthritic Sign** | **Group** | **Mean** | **Pr > t** | | **Osteoarthritic Sign** | **Diet** | **Mean** | **Pr > t** |
| Rising from rest | A | -0.463 | <.0001 | | Running | A | -0.579 | <.0001 |
| | B | -0.633 | <.0001 | | | B | -0.558 | <.0001 |
| | C | -0.518 | <.0001 | | | C | -0.605 | <.0001 |
| Limping | A | -0.489 | 0.0003 | | Walking | A | -0.294 | 0.0358 |
| | B | -0.588 | <.0001 | | | B | -0.643 | <.0001 |
| | C | -0.681 | <.0001 | | | C | -0.595 | <.0001 |
| Stiffness | A | -0.255 | 0.0420 | | Stair Climbing | A | -0.419 | 0.0024 |
| | B | -0.483 | <.0001 | | | B | -0.489 | 0.0002 |
| | C | -0.589 | <.0001 | | | C | -0.689 | <.0001 |
| Soreness | A | -0.810 | <.0001 | | Jumping | A | -0.571 | 0.0003 |
| | B | -0.920 | <.0001 | | | B | -0.479 | 0.0011 |
| | C | -0.926 | <.0001 | | | C | -0.773 | <.0001 |
| Lagging behind on walks | A | -0.657 | <.0001 | | Playing | A | -0.606 | 0.0002 |
| | B | -0.531 | 0.0014 | | | B | -0.571 | 0.0003 |
| | C | -0.448 | 0.0094 | | | C | -0.694 | <.0001 |
| Pain | A | -0.684 | 0.0002 | | Lameness | A | -0.484 | 0.0045 |
| | B | -0.571 | 0.0009 | | | B | -0.778 | <.0001 |
| | C | -0.667 | 0.0010 | | | C | -0.667 | <.0001 |
| Aggression | A | -0.750 | 0.0234 | | Activity Level | A | -0.409 | 0.0009 |
| | B | -1.000 | 0.0025 | | | B | -0.704 | <.0001 |
| | C | -1.000 | 0.0751 | | | C | -0.551 | <.0001 |

| | | | | | | | | |
|---|---|---|---|---|---|---|---|---|
| The above "p" values refer to the mean change from day 0 to day 90. | | | | | | | | |

Dogs consuming higher concentrations of n-3 fatty acids were reported to have more significant improvement in osteoarthritic condition and more significant reduction in the progression of osteoarthritis than those dogs receiving the lowest dosage, based on veterinarians clinical assessments (Table 16). There was no significant difference in improvement in osteoarthritic condition or reduction in the progression of osteoarthritis between the group receiving medications and / or supplements and the non-medicated group (Table 17). This indicates that the therapeutic diets work synergistically with other therapies or at least not withstanding other therapies by providing additional benefit to dogs suffering from osteoarthritis.

An extremely low incidence of adverse reactions or side effects were reported among dogs participating in this study. Only five dogs out of the 215 animals assigned to food were reported to have diarrhea and vomiting, which could possibly be attributed to consuming one of dietary variables. Similar incidence of adverse reactions or side effects were reported for those dogs consuming the therapeutic diets in the previous two studies discussed (1/88 and 1/26 for examples 1 and 2 respectively.

**TABLE 15.**

| **Difference in Pet Owners Frequency Rating (day 0-90)** | | | | | | | | |
|---|---|---|---|---|---|---|---|---|
| **Osteoarthritic Sign** | **Group** | **Mean** | **Pr > t** | | **Osteoarthritic Sign** | **Diet** | **Mean** | **Pr > t** |
| | A | -0.370 | <.0001 | | | A | -0.239 | <.0165 |
| Rising from rest | B | -0.467 | <.0001 | | Limping | B | -0.365 | <.0001 |
| | C | -0.509 | <.0001 | | | C | -0.396 | <.0001 |
| | A | -0.098 | 0.2929 | | Lagging | A | -0.571 | <.0001 |
| Stiffness | B | -0.373 | <.0001 | | Behind on | B | -0.643 | <.0001 |
| | C | -0.421 | <.0001 | | Walks | C | -0.500 | 0.0004 |
| | A | -0.381 | 0.0146 | | | A | -0.417 | 0.0536 |
| Soreness | B | -0.680 | <.0001 | | Aggression | B | -0.467 | 0.0175 |
| | C | -0.821 | <.0001 | | | C | -0.167 | 0.5741 |
| | A | -0.447 | 0.0004 | | | A | -0.206 | 0.0911 |
| Running | B | -0.395 | 0.0009 | | Walking | B | -0.558 | <.0001 |
| | C | -0.477 | <.0001 | | | C | -0.447 | 0.0002 |
| | A | -0.357 | 0.0027 | | | A | -0.302 | 0.0069 |
| Jumping | B | -0.354 | 0.0015 | | Stair Climbing | B | -0.348 | 0.0014 |
| | C | -0.467 | <.0001 | | | C | -0.457 | <.0001 |
| | A | -0.455 | 0.0013 | | Impaired | A | -0.250 | 0.0643 |
| Playing | B | -0.297 | 0.0238 | | Mobility | B | -0.436 | 0.0005 |
| | C | -0.667 | 0.0010 | | | C | -0.667 | <.0001 |

**TABLE 16.**

| **Progression of Osteoarthritic Condition** | | | | | | **Overall Change in Osteoarthritic Condition** | | | | |
|---|---|---|---|---|---|---|---|---|---|---|
| **Diet** | **N** | **Mean** | **P** | | | **Diet** | **N** | **Mean** | **P** | |
| A | 55 | 2.327 | 0.2891 | A vs B | | A | 54 | 3.148 | 0.1675 | A vs B |
| B | 62 | 2.177 | 0.1619 | B vs C | | B | 62 | 2.871 | 0.0787 | B vs C |
| C | 59 | 1.983 | 0.0168 | A vs C | | C | 59 | 2.525 | 0.0024 | A vs C |

**TABLE 17.**

| **Progression of Osteoarthritic Condition** | | | | | | **Overall Change in Osteoarthritic Condition** | | | | |
|---|---|---|---|---|---|---|---|---|---|---|
| **Diet** | **Medicated** | **N** | **Mean** | **P** | | **Diet** | **Medicated** | **N** | **Mean** | **P** |
| A | No | 22 | 2.273 | 0.6665 | | A | No | 21 | 3.143 | 0.9770 |
| A | Yes | 33 | 2.364 | | | A | Yes | 33 | 3.152 | |
| B | No | 23 | 2.130 | 0.7109 | | B | No | 23 | 2.696 | 0.3247 |
| B | Yes | 39 | 2.205 | | | B | Yes | 39 | 2.974 | |
| C | No | 28 | 2.071 | 0.4003 | | C | No | 28 | 2.750 | 0.1285 |
| C | Yes | 31 | 1.903 | | | C | Yes | 31 | 2.323 | |

## Claims

1. Use of EPA in the manufacture of a medicament for preventing or diminishing the degenerative process in joint cartilage in a dog having osteoarthritis or in the manufacture of a medicament for deceasing the likelihood of a dog developing osteoarthritis, wherein the medicament comprises EPA at a concentration of at least 0.2% by weight and the medicament, is to be administered to the dog in amount providing at least 27.5 mg EPA/kg body weight.

2. A use according to claim 1, wherein the medicament comprises a diet comprising EPA in an amount of at least 0.2% by weight

3. A use according to any of claims 1 or 2, wherein the medicament comprises EPA at a concentration of at least 0.3% by weight.

4. A use according to any of claims 1.3, wherein the medicament comprises a ratio of omega-6 fatty acids to omega-3 fatty acids of 0.2 to 1.1.

5. A use according to any of claims 1-3, wherein medicament comprises a ratio of omega-6 fatty acids to EPA of 1.0 to 12.5.

6. A use according to any of claims 1-3, wherein the medicament comprises an food composition, a treat or a supplement.

## Patentansprüche

1. Verwendung von EPA bei der Herstellung eines Arzneimittels zur Verhinderung oder Abschwächung des degenerativen Prozesses bei Gelenkknorpel bei einem Hund mit Osteoarthritis oder bei der Herstellung eines Arzneimittels zur Verminderung der Wahrscheinlichkeit, dass ein Hund Osteoarthritis entwickelt, wobei das Arzneimittel EPA in einer Konzentration von mindestens 0,2 Gew.% umfasst und das Arzneimittel dem Hund so in einer Menge zu verabreichen ist, dass mindestens 27,5 mg EPA/kg Körpergewicht bereitgestellt werden.

2. Verwendung nach Anspruch 1, wobei das Arzneimittel eine Diät umfasst, die EPA in einer Menge von mindestens 0,2 Gew.% umfasst.

3. Verwendung nach einem der Ansprüche 1 oder 2, wobei das Arzneimittel EPA in einer Konzentration von mindestens 0,3 Gew.% umfasst.

4. Verwendung nach einem der Ansprüche 1 bis 3, wobei das Arzneimittel ein Verhältnis von Omega-6-Fettsäuren zu Omega-3-Fettsäuren von 0,2 bis 1,1 aufweist.

5. Verwendung nach einem der Ansprüche 1 bis 3, wobei das Arzneimittel ein Verhältnis von Omega-6-Fettsäuren zu EPA von 1,0 bis 12,5 aufweist.

6. Verwendung nach einem der Ansprüche 1 bis 3, wobei das Arzneimittel eine Futterzusammensetzung, ein Leckerli oder ein Ergänzungsmittel umfasst.

## Revendications

1. Utilisation d'acide éicosapentaénoïque (EPA) dans la fabrication d'un médicament pour prévenir ou diminuer le processus dégénératif dans le cartilage articulaire chez un chien ayant de l'ostéoarthrite ou dans la fabrication d'un médicament pour décéder la probabilité d'un chien à développer de l'ostéoarthrite, dans laquelle le médicament comprend de l'EPA à une concentration d'au moins 0,2 % en poids et le médicament est à administrer au chien dans une quantité fournissant au moins 27,5 mg d'EPA / kg de poids corporel.

2. Utilisation selon la revendication 1, dans laquelle le médicament comprend un régime comprenant de l'EPA dans une quantité d'au moins 0,2 % en poids.

3. Utilisation selon l'une quelconque des revendications 1 ou 2, dans laquelle le médicament comprend de l'EPA à une concentration d'au moins 0,3 % en poids.

4. Utilisation selon l'une quelconque des revendications 1 à 3, dans laquelle le médicament comprend un rapport d'acides gras omega-6 sur acides gras omega-3 de 0,2 à 1,1.

5. Utilisation selon l'une quelconque des revendications 1 à 3, dans laquelle le médicament comprend un rapport d'acides gras omega-6 sur EPA de 1,0 à 12,5.

6. Utilisation selon l'une quelconque des revendications 1 à 3, dans laquelle le médicament comprend une composition alimentaire, une gâterie ou un complément.
